# EUROPEAN PATENT APPLICATION

(11) **EP 0 950 356 A1**
(43) Date of publication of application: **20.10.1999**
(21) Application number: 98201233.8
(22) Date of filing: 17.04.1998
(51) Int. Cl.: A23K 1/16, A23L 1/054, A23L 2/02, C12P 19/04

(54) **Use of polysaccharides derived from yeasts as technological coadjuvants in the production of preserved foods**

(71) Applicant: Farmint Group Holding S.A., 1413 Luxembourg (LU)
(72) Inventor: Lazzari, Fabrizio, 43100 Parma (IT)
(74) Representative: Ferreccio, Rinaldo

(57) **Abstract**

Use of polysaccharides of the class of glucans or mannans obtained from yeasts or fungi, particularly *Saccharomyces cerevisiae*, as technological coadjuvants in the preparation of preserved food products such as, for example, vegetable or fruit juices and concentrates and powdered, dried or lyophilized extracts of vegetables or fruit, or preserved animal products such as cooked meats, charcuterie and sausages; for the production of tomato concentrates in particular a method is used which comprises a step for preheating the previously chopped tomato, and in which the aforementioned polysaccharides are added to the chopped tomato before the preheating step in quantities variable from 0.3 to 8% by weight relative to the weight of tomato.

## Description

The present invention relates in general to the use of polysaccharides derived from yeasts and/or fungi in the food industry.

In particular, the invention relates to the use of polysaccharides of the glucan and mannan classes derived from yeasts and/or fungi as technological coadjuvants in the production of preserved foods such as juices, sauces and concentrates of vegetables (for example, tomatoes) and fruit, cooked meats, charcuterie and sausages.

The food industry is constantly seeking natural substances which can be used in its processes as technological coadjuvants for wholly or partially replacing currently-used additives of non-natural origin.

In the particular case in question, research efforts have been directed towards the identification of substances of natural origin which might improve the methods of preparing preserved foods such as fruit or vegetable juices, sauces and extracts, cooked meats, charcuterie and sausages, as well as the preservability of the final products.

Of the substances tested, polysaccharides derived from yeasts and/or fungi have given results which are extremely satisfactory and even surprising from certain points of view.

The polysaccharides in question are constituted by glucans and mannans which are the principal components of the cell walls of yeasts and fungi; the polysaccharides which are most interesting from the point of view of the food industry are those derived from *Saccharomyces cerevisiae* since this is a micro-organism which has been used by man in food for centuries and which therefore offers the best guarantee of being harmless.

The cell walls of *Saccharomyces cerevisiae* are composed mainly of glucan which in turn is constituted by a main chain of glucose units joined by β(1-3) glucoside bonds with a low degree of inter- and intramolecular branching by means of β(1- 6) glucoside bonds.
The polysaccharides contained in the walls of *Saccharomyces cerevisiae* can be obtained by various methods such as, for example, those described in US patents 5 082 936 and 3 867 554, and in Italian patent application MI96A000681.

The use of these substances as thickeners for food preparations such as creams, ices, sauces, etc. has been proposed because of their ability to absorb and retain large quantities of water and to increase viscosity.

Moreover, when added to substantially liquid food products, they cause these products to produce a sensation of a fatty consistency when tasted, even though these polysaccharides are substantially fat free.

It has now surprisingly been found that the above- mentioned polysaccharides can be used as technological coadjuvants in methods of preparing preserved foods on which they confer improved organoleptic characteristics and preservability.

They are preferably added to the foods to be treated in quantities of between 0.1 and 8%, advantageously between 0.2 and 2%.

Non-limiting examples of preserved foods in the preparation of which the aforementioned polysaccharides can be used are the following: juices and concentrates of fruits (peaches, apricots, pears, apples, oranges, etc.) and of vegetables (tomatoes, carrots, etc.) tomato concentrates of various types (semi-concentrates, double, triple and sextuple tomato concentrates), pure tomato concentrates in powder form, peeled whole or chopped tomatoes with juice, cooked meats, charcuterie and sausages such as ham, salami, etc.

Particularly good results have been obtained with the use of polysaccharides derived from *Saccharomyces cerevisiae* obtained by a method which constitutes a further aspect of the present invention.

This method comprises the steps of:
- preparing a suspension of *Saccharomyces cerevisiae* yeast in a 0.1-0.3N solution of a strong acid to give a solids content of 20-25% and a pH of from 2 to 5,
- maintaining stirring at 75-80°C for 30-90′,
- separating the solid component of the suspension,
- resuspending the solid component in a 25-30% NaCl solution to give a solids content of 25-30% and leaving it to rest for 12-15 hours,
- separating the solid component and washing it with water,
- resuspending the solid component in a 0.1-0.3N solution of an alkali hydroxide to give a solids content of 25-30%,
- maintaining stirring at 75-80°C for 30-90′,
- neutralizing with concentrated HCl and recovering the solid component by separating it from the supernatant liquid.

Preferably, the above-mentioned strong acid is HCl and the above-mentioned alkali hydroxide is NaOH.

The above-mentioned steps for separating the solid component are carried out by centrifuging or, preferably, by filtration.

To increase the purity of the polysaccharides obtained by the method described above, the solid component separated after the treatment with the NaCl solution and washed with water may be washed again with an ethyl alcohol/ethyl ether mixture.

This ensures that any protein and fatty components and other organic impurities are eliminated to a maximum extent.

Again for this purpose, it is useful to subject the solid component recovered after neutralization with HCl to washing first with water and then with an ethanol/ethyl ether mixture.

The solid residue obtained from the last separation may be resuspended in water to give a suspension with a 40% solids content which is then dried, for example, by means of drum driers.

The final dried product (0.4-0.6% moisture) is in the form of a powder of variable particle size or in flakes of considerably attenuated colour and flavour in comparison with the original yeast. A 1% aqueous suspension of the product has a pH of about 4.7. When the above-mentioned washings with water and with an ethanol/ethyl ether mixture are carried out, the impurities content of the final product is less than 1%.

The method described above gives a product with optimal purity and organoleptic characteristics without involving the need for hydrolysis carried out with large volumes of water which is typical of methods of the prior art. In fact, suspensions having a solids content of at least 20% are used, whereas known methods use suspensions with solids contents of 3-10%. This leads to considerable savings in the management of the reaction and separation plants.

Moreover, the soluble fraction obtained upon completion of the first hydrolysis (the acid hydrolysis) can be recovered and constitutes a source of proteins, nucleic acids and nucleotides which is useful, for example, in the livestock and fodder industries. The same applies to the soluble fraction separated upon completion of the second hydrolysis. This avoids the problems of environmental pollution due to the disposal of waste products since every fraction separated is re-used in some way and no actual waste product is in fact generated.

The advantages of the present invention will become clearer from the non-limiting examples given below.

### EXAMPLE 1

10kg of lyophilized live bakers' yeast (*Saccharomyces cerevisiae*) was suspended with 30 litres of a 0.2N HCl solution giving a suspension with a solids content of 25% and a pH of 4.3.

The suspension was heated to 78°C with stirring for 60′, was cooled to ambient temperature and was filtered with a porous plate filter in order to separate the solid component from the liquid component.

The solid component was resuspended with 30 litres of 25% NaCl and left to rest for 15 hours. The suspension was then re-filtered to remove the supernatant liquid.

The residue was washed with water to achieve a negative chloride reaction in the washing water and then with a 1:1 ethanol/ethyl ether mixture.

The residue was then resuspended with 30 litres of a 0.2N NaOH solution (final solids content about 25%) and the resulting suspension was kept at 75°C for 30′ with stirring.
After cooling to ambient temperature, the suspension was neutralized with 37% HCl and filtered to remove the supernatant liquid.

The residue was washed with water and then with a 1:1 ethanol/ethyl ether mixture.

The residue was then resuspended in water to give a suspension with a 40% solids content which, finally, was dried by a drum drier.

The final product had 0.5% moisture and was in the form of a powder of almost imperceptible odour and flavour. The impurities content was about 0.8%. A 1% aqueous suspension of the product had a pH of 4.7. The activity of the water of the product was less than 0.4.

### EXAMPLE 2

### Preparation of tomato juice

Tomato juice (this term defining preserved tomato juice, partially evaporated to a solids content of about 8-10%) was prepared by a "cold break" technique, that is, a technique which, after preliminary steps for the washing, grading and chopping of the tomatoes, provides for a step of pre-heating to 65-75°C prior to concentration.

Before the pre-heating step was carried out, the polysaccharide product obtained in accordance with Example 1 was added to the chopped tomatoes in a quantity equal to 0.5% of the total weight of tomatoes.

The step of pre-heating to 70°C was then carried out in accordance with the "cold break" technique and the subsequent concentration step was carried out by a bubble concentrator.

Upon completion, a juice (sample C) was obtained, the physical, chemical and organoleptic characteristics of which are given in Table 1 below.

The preparation of the tomato juice was repeated in identical manner but without the addition of the polysaccharides before pre-heating. The juice indicated "sample A" in Table 2 was obtained.

Finally, a juice (sample B) was prepared by the same method as for sample A but with the pre-heating step carried out by the "hot break" technique at a temperature of 88°C.

**TABLE 1**

| **Sample** | | A | B | C |
|---|---|---|---|---|
| Preservation state | | Normal | Normal | Normal |
| Colour | | characteristic | characteristic | characteristic |
| Odour | | caramelized | slightly caramelized | characteristic fresh tom. |
| Flavour | | slightly caramelized | slightly caramelized | characteristic fresh tom. |
| Soluble Solids Content (SSC) | % | 8.20 | 8.10 | 8.20 |
| Total Solids Content (TSC) | % | 8.81 | 9.20 | 8.93 |
| Total Solids Content - Added Chlorides (TSC - AC) | % | 8.81 | 9.20 | 8.93 |
| Total chlorides | % | 0.15 | 0.15 | 0.15 |
| Added Chlorides (as NaCl) | % | absent | absent | absent |
| Reducing sugars | % | 4.29 | 4.38 | 4.38 |
| Reducing sugars % of (TSC - AC) | | 48.69 | 47.61 | 49.05 |
| Reducing sugars after inversion | % | 4.29 | 4.38 | 4.38 |
| Reduc. sugars after inv. % of (TSC - AC) | | 48.69 | 47.61 | 49.05 |
| Acidity in citric acid monohydr. | % | 0.46 | 0.46 | 0.46 |
| Acidity total % of (TSC - AC) | | 5.22 | 5.00 | 5.15 |
| pH | | 4.36 | 4.35 | 4.36 |
| Bostwick at SSC = T.Q. | | 12.7 | 4.1 | 4.4 |
| Gardner colour at SSC = T.Q. | a/b | 2.20 | 2.27 | 2.33 |
| Syneresis after 30′ | | positive | negative | negative |

As can be seen from Table 1, Sample C had markedly better organoleptic characteristics than Samples A and B (see in particular, the odour, flavour and Gardner colour).

The Bostwick consistency, syneresis and the ratio soluble solids content/(total solids content - added chlorides) of Sample C practically coincided with those of Sample B although the latter was obtained by a much more expensive technique and with higher production costs.

It is interesting to note that no increase in the sugars ratio was noted in sample C after inversion of the sugars, which confirms that the polysaccharides added were not hydrolysed to any extent.

Similar favourable results were obtained with the addition of the polysaccharides in the preparation of carrot juice and various vegetable juices.

### EXAMPLE 3

### Preparation of a double tomato concentrate

Starting with the three tomato juices (A, B and C) produced in the previous example, respective double tomato concentrates were produced by concentration in a bubble concentrator. In the case of juice C, the polysaccharides obtained in accordance with Example 1 were added in a quantity of 1% by weight relative to the weight of the tomato juice, before the concentration was carried out.

The results of physical, chemical and organoleptic analysis carried out on the three final double concentrates are summarized in Table 2 below.

**TABLE 2**

| **Sample** | | A | B | C |
|---|---|---|---|---|
| Preservation state | | Normal | Normal | Normal |
| Colour | | characteristic | characteristic | characteristic |
| Odour | | caramelized | slightly caramelized | characteristic fresh tom. |
| Flavour | | slightly caramelized | characteristic | characteristic fresh tom. |
| Soluble Solids Content (SSC) | % | 28.60 | 28.00 | 28.40 |
| Total Solids Content (TSC) | % | 29.97 | 30.34 | 30.19 |
| Total Solids Content - Added Chloride (TSC - AC) | % | 29.97 | 30.34 | 30.19 |
| Total Chlorides | % | 0.47 | 0.47 | 0.47 |
| Added Chlorides (as NaCl) | % | absent | absent | absent |
| Reducing sugars | % | 14.71 | 14.93 | 14.93 |
| Reducing sugars % of (TSC - AC) | | 49.08 | 49.21 | 49.45 |
| Reducing sugars after inversion | % | 14.71 | 14.93 | 14.93 |
| Reduc. sugars after inv. % of (TSC- AC) | | 49.08 | 49.21 | 49.45 |
| Acidity in citric acid monohydr. | % | 1.82 | 1.68 | 1.75 |
| Acidity total % of (TSC - AC) | | 6.07 | 5.54 | 5.80 |
| pH | | 4.33 | 4.31 | 4.32 |
| Bostwick at SSC = 12.00% | | 12.90 | 3.80 | 3.80 |
| Gardner colour at SSC = 12.00% | a/b | 2.08 | 2.10 | 2.16 |
| Syneresis after 30′ | | positive | negative | negative |

In this case also, a considerable improvement in the organoleptic characteristics (odour, flavour, Gardner colour) was noted for the double concentrate C in comparison with both of Samples A and B. It was also noted that the Bostwick consistency of Sample C was identical to that of Sample B, although the preheating step was carried out by the cold break technique instead of by the hot break technique.

### EXAMPLE 4

### Preparation of a triple tomato concentrate

The concentration of the double concentrates A and C was continued to produce the respective triple concentrates (a triple concentrate was not prepared from the double concentrate B, however, because of the technical impossibility of preparing triple concentrates by hot break techniques).

In this case also, physical chemical and organoleptic analysis showed the superiority of the product to which polysaccharides according to the invention had been added, as shown in Table 3.

**TABLE 3**

| **Sample** | A | C |
|---|---|---|
| Preservation state | Normal | Normal |
| Colour | slightly dark | characteristic fresh tom. |
| Odour | slightly caramelized | characteristic fresh tom. |
| Flavour | slightly burnt | characteristic fresh tom. |
| Soluble Solids Content (SSC) | 37.30 | 37.10 |
| Total Solids Content (TSC) | 38.87 | 39.23 |
| Total Solids Content - Added Chlorides (TSC - AC) | 38.87 | 39.23 |
| Total Chlorides | 0.64 | 0.64 |
| Added Chlorides (as NaCl) | absent | absent |
| Reducing sugars | 19.25 | 19.62 |
| Reducing sugars % of (TSC - AC) | 49.52 | 50.01 |
| Reducing sugars after inversion | 19.25 | 19.62 |
| Reduc. sugars after inv. % of (TSC - AC) | 49.25 | 50.01 |
| Acidity in citric acid monohydr. | 2.59 | 2.56 |
| Acidity total % of (TSC - AC) | 6.66 | 6.53 |
| pH | 4.33 | 4.33 |
| Bostwick at SSC = 12.00% | 11.50 | 5.40 |
| Gardner colour at SSC = 12.00% | 2.10 | 2.19 |
| Syneresis after 30′ | negative | negative |

### EXAMPLE 5

### Preparation of pure tomato concentrate in powder form

The following three tests were performed:
1) The double tomato concentrate A (cold break) of Example 3 was mixed in a 1:1 ratio with the triple concentrate C of Example 4 and the mixture obtained was diluted with water to a soluble solids content of 20-22%.
2) The double tomato concentrate B (hot break) of Example 3 was mixed in a 1:1 ratio with the triple concentrate C of Example 4 and the mixture obtained was diluted with water to a soluble solids content of 20- 22%.
3) The double tomato concentrate C (cold break) of Example 3 was mixed in a 1:1 ratio with the triple concentrate C of Example 4 and, after the mixture obtained had been diluted with water to a soluble solids content of 20-22%, polysaccharides according to Example 1 were added in a quantity of 1% by weight relative to the weight of the mixture thus diluted. The three mixtures thus prepared and diluted were subjected to drying by means of a drum drier to give a tomato concentrate in powder form.

The drying was carried out at the usual temperatures for Tests 1 and 2 whereas, in the case of Test 3, it was possible to achieve drying at a temperature 15- 20°C lower than the usual drying temperatures, for a given production yield.

The results of the physical chemical and organoleptic analysis are given in Table 4 below.

**TABLE 4**

| Test No. | | 1 | 2 | 3 |
|---|---|---|---|---|
| Preservation state | | Normal | Normal | Good |
| Colour | | red-brown | slightly brown | characteristic red |
| Odour | | burnt | slightly caramelized | characteristic fresh |
| Flavour | | burnt | slightly caramelized | characteristic fresh |
| Total Solids Content (TSC) | % | 96.89 | 97.12 | 97.42 |
| Total Solids Content - Added Chlorides (TSC - AC) | % | 96.89 | 97.12 | 97.42 |
| Total Chlorides | % | 1.61 | 1.61 | 1.61 |
| Added Chlorides (as NaCl) | % | absent | absent | absent |
| Reducing sugars | % | 46.82 | 47.58 | 49.52 |
| Reducing sugars % of (TSC - AC) | | 48.32 | 48.99 | 50.83 |
| Reducing sugars after inversion | % | 46.82 | 47.58 | 49.52 |
| Reduc. sugars after inv. % of (TSC - AC) | | 48.32 | 48.99 | 50.83 |
| Acidity in citric acid monohydr. | % | 6.93 | 6.86 | 6.93 |
| Acidity total % of (TSC - AC) | | 7.15 | 7.06 | 7.11 |
| pH at SSC = 12.00% | | 4.38 | 4.35 | 4.34 |
| Blotter test at SSC = 12.00% | | off scale | 23.90 | 20.30 |
| Gardner colour at SSC = 12.00% | a/b | 2.10 | 2.16 | 2.16 |
| Rehydration test | | poor | good | very good |

The qualitative improvement found for the product produced by Test 3 was particularly clear with regard to the organoleptic characteristics, the sugars ratio and the Gardner colour.

The production yields were better than those of Tests 1 and 2.

The product produced by Test 3 had optimal rehydration characteristics; these characteristics were evaluated by the following test.

250 ml of water was added to 30 g of each sample produced by Tests 1, 2 and 3 and the mixtures thus obtained were brought to boiling point after which they were poured onto white plates to check for the presence of lumps and for syneresis.

The sample obtained by Test 3 dissolved perfectly without leaving lumps and did not show any syneresis. The sample obtained by Test 1, on the other hand, had clear syneresis and syneresis was also shown to a certain, though lesser, extent by the sample produced by Test 2.

Moreover, the shelf life at ambient temperature of the product produced by Test 3 was markedly longer than that of the products obtained by Tests 1 and 2.

To check preservability at ambient temperature, the above-mentioned products were packed in the usual combined aluminium/polypropylene bags and were kept at ambient temperature for 60 days.

After 30 days had elapsed, the samples produced by Tests 1 and 2 were compacted and hard whereas the sample produced by Test 3 showed no tendency to harden or to become compacted even after 60 days.

This latter fact indicates a lesser tendency of the sample produced in accordance with Test 3 to absorb atmospheric moisture.

### EXAMPLE 6

### Preparation of peeled, chopped tomatoes (tomato pulp)

Peeled, chopped tomatoes in juice with a final residue of about 7% were prepared with the use of a tomato juice with a soluble solids content of 10-12%.

Peeled, chopped tomatoes were then prepared in the same manner with the same tomato juice with 10-12% soluble solids content, but with the addition of polysaccharides produced in accordance Example 1 in a quantity of 1% by weight relative to the weight of the juice.

The addition of the polysaccharides to the juice led to organoleptic, physical and chemical improvements which can be inferred from Table 5 below.

**TABLE 5**

| Test | Conventional | With polysaccharides |
|---|---|---|
| preservation state | normal | normal |
| colour | red | bright red |
| odour | characteristic | characteristic, intense |
| flavour | characteristic | characteristic |
| Soluble Solids Content % | 7.00 | 7.10 |
| pH | 4.30 | 4.30 |
| Syneresis after 30′ | present | absent |

In addition to an improvement in organoleptic characteristics and in syneresis, improved mixing of the finished product was noted and led to covering of any defects such as unripe or poorly matured pieces of tomato or green parts, etc.

The same improvements found with the use of the polysaccharides derived from yeasts and/or fungi in the preparation of juices and tomato concentrates were also found when these polysaccharides were used as technological coadjuvants in the production of juices and pulps of pears, apples, apricots and oranges, in the production of all preserved fruits or vegetables comprising solids in liquid preparations and extracts of fruit or vegetables produced by drying or lyophilization.

In addition to qualitative improvements in the preserved products, with the use of the aforementioned polysaccharides a significant reduction in production costs is achieved, particularly with regard to energy consumption.

### EXAMPLE 7

### Preparation of cooked shoulder of pork

A batch of pork shoulder joints from the same slaughterhouse was divided into two lots A and B.

Lot A was subjected to conventional processing comprising trimming of the joints and treatment thereof with a mixture containing salt and suitably metered spices.

The shoulder joints of lot B were also trimmed and treated with a mixture which, in addition to the salt and spices which were present in the same quantities as for lot A, also contained polysaccharides produced in accordance with Example 1 in a quantity equal to 1.5% by weight relative to the weight of the meat for joints weighing less than 2.5 kg (B₁) and 3% for joints weighing more than 2.5 kg (B₂).

The salted fresh shoulders were placed in cooling chambers to rest at 0°-4°C for 3 days after being passed through a churn (a mixer). They were then salted a second time with the same mixtures as described above and returned to the chamber to rest at 0°-4°C for 7 days.

After the non-absorbed or excess saline mixture had been carefully removed, the shoulders were packed in previously prepared bovine bladders.

The shoulders were then tied and the bladders were perforated before the shoulders were kept at 19-20°C in a drier for three days.

When this time had elapsed, the shoulders were cooked by bringing the temperature to a value of 70°C in the centre of the shoulders and cooling rapidly to below 50°C in a douche.

The shoulders were then kept at 0°-4°C in a cooling chamber for 12 hours.

After packaging under vacuum in suitable bags, the shoulders were pasteurized for 10′ at 88°-90°C, and then cooled to below 25°C.

The cooked shoulders of lot A showed the presence of a greater quantity of saline solution and fat inside the vacuum pack in comparison with those of lot B which also had a much more pleasing overall appearance.

Organoleptic evaluation to compare the two lots of cooked shoulder showed a decidedly better taste and tenderness for the shoulders of lot B, particularly those of lot B₁.

### EXAMPLE 8

### Preparation of salami of the "felino" type

Pork from a single slaughterhouse, suitably chopped and divided into two lots A and B of the same weight was used.

Lot A was supplemented with suitably metered ingredients conventionally used in the preparation of felino salami (salt, spices etc.).

Lot B was divided into two equal portions B₁ and B₂ which were supplemented with the same ingredients as lot A in the same quantities and with quantities of 1% and 3% by weight, relative to the weight of the meat, of polysaccharides obtained in accordance with Example 1, respectively.

The various lots were suitably mixed with a mixer and placed in a cooling chamber to rest at 5°-7°C overnight. They were then packed in previously prepared pig guts and tied manually before the salami thus produced were sent to the drying room where they were kept in the temperature, humidity and ventilation conditions provided for by good production standards.

The salami were then transferred to maturing rooms where they remained until maturing was complete in accordance with the conditions provided for by good production standards.

The various production stages were monitored carefully and a considerable reduction in the times required for drying and blooming, with a consequent reduction in maturing times, was found for the samples B, particularly B₂.

The salami produced from lot B were softer on the palate and the drop in weight upon completion of the maturing was about 3% less than that recorded for the salami produced from lot A for B₁, and about 6.5% less for B₂.

## Claims

1. Use of polysaccharides of the class of glucans or mannans obtained from yeasts or fungi as technological coadjuvants in the preparation of preserved food products.

2. Use according to Claim 1 in which the polysaccharides are obtained from *Saccharomyces cerevisiae*.

3. Use according to Claim 1 or Claim 2, in which the preserved food products comprise juices and concentrates of vegetables or fruit, powdered, dried or lyophilized extracts of vegetables or fruit, and preserved animal products such as cooked meats, charcuterie and sausages.

4. Use according to Claim 3, in which the vegetable juices and concentrates are tomato juices and concentrates, respectively.

5. A method of producing tomato concentrates, comprising a step in which the previously chopped tomato is preheated, characterized in that polysaccharides of the class of glucans or mannans obtained from yeasts or fungi are added to the chopped tomato before the preheating step in quantities variable from 0.3 to 8% by weight relative to the weight of the tomato.

6. A method according to Claim 5, in which the polysaccharides are obtained from *Saccharomyces cerevisiae*.

7. A method according to Claim 6, in which the polysaccharides are added in quantities variable from 0.5 to 5% by weight relative to the weight of the chopped tomato.

8. A method of producing polysaccharides such as glucans or mannans from bakers' yeast (*Saccharomyces cerevisiae*), comprising the steps of:
a) preparing a suspension of baker's yeast in a 0.1- 0.3N solution of a strong acid to give a solids content of 20-25% and a pH of from 2 to 5,
b) maintaining stirring at 75-80°C for 30-90′,
c) separating the solid component of the suspension from the liquid component,
d) resuspending the solid component in a 25-30% NaCl solution to give a solids content of 25-30% and leaving it to rest for 12-15 hours,
e) separating the solid component and washing it with water,
f) resuspending the solid component in a 0.1-0.3N solution of an alkali hydroxide to give a solids content of 25-30%,
g) maintaining stirring at 75-80°C for 30-90′,
h) neutralizing with concentrated HCl and recovering the solid component by separating it from the supernatant liquid.

9. A method according to claim 8, wherein said strong acid is HCl and said alkali hydroxide is NaOH.

10. A method according to any one of Claims 8 and 9, in which the steps for separating the solid component are carried out by filtration.

11. A method according to any one of Claims 8 to 10, further comprising the step of washing the solid component obtained upon completion of step e) with an ethanol/ethyl ether mixture.

12. A method according to Claim 11, further comprising the step of washing the solid component obtained upon completion of step h first with water and then with an ethanol/ethyl ether mixture.

13. Use according to any one of Claims 1 to 4, in which the polysaccharides are obtained by the method according to any one of Claims 8-12.

14. A method according to any one of Claims 5 to 7, in which the polysaccharides are obtained by means of the method according to any one of Claims 8-12.

15. Use of the liquid component separated in step c) of the method according to Claim 8 as a nutrient source for the livestock industry and as an ingredient for fodder compositions.
